# EUROPEAN PATENT APPLICATION

(11) **EP 3 124 544 A1**
(43) Date of publication of application: **01.02.2017**
(21) Application number: 15768451.5
(22) Date of filing: 27.03.2015
(51) Int. Cl.: C08L 67/04, C12N 1/21, C12N 15/09, C12P 7/42

(54) **POLYESTER RESIN COMPOSITION, COMPACT FORMED FROM SUCH RESIN COMPOSITION, AND METHOD FOR MANUFACTURING SUCH COMPACT**

(30) Priority: 28.03.2014 JP 2014067675
(71) Applicant: Kaneka Corporation, Osaka-shi, Osaka 530-8288 (JP)
(72) Inventor: MINAMI, Tetsuya, (JP); KOBAYASHI, Shingo, (JP); AOKI, Rina, (JP); SUZUKI, Noriyuki, (JP)
(74) Representative: Vossius & Partner Patentanwälte Rechtsanwälte mbB
(86) International application number: PCT/JP2015/001794
(87) International publication number: WO 2015/146194

(57) **Abstract**

The purpose of the present invention is to improve processability in the melt-molding by improving the slowness of crystallization that is a disadvantage of polyhydroxyalkanoate, which is slow to crystallize, and to improve processing speed. Provided is a polyester resin composition including a first polyhydroxyalkanoate and a second polyhydroxyalkanoate, wherein the melting point of the second polyhydroxyalkanoate in the polyester resin composition is observed at a temperature lower than the melting point measured for the second polyhydroxyalkanoate alone.

SEQUENCE LISTING

<110>  KANEKA Corporation

<120>  Polyester resin composition, and molded article formed therefrom and
its production process

<130>  Z2467 EP

<140>  EP 15 76 8451.5
<141>  2015-03-28

<150>  JP2014-067675
<151>  2014-03-28

<160>  30

<170>  PatentIn version 3.5

<210>  1
<211>  32
<212>  DNA
<213>  Artificial Sequence

<220>
<223>  primer

<400>  1
gcgcatttaa atccggacct tcgtgcggct ca                                    32


<210>  2
<211>  20
<212>  DNA
<213>  Artificial Sequence

<220>
<223>  primer

<400>  2
gaggactcct gatcgtgtga                                                  20


<210>  3
<211>  40
<212>  DNA
<213>  Artificial Sequence

<220>
<223>  primer

<400>  3
tcacacgatc aggagtcctc agtcgggcag caccaatgcg                            40


<210>  4
<211>  32
<212>  DNA
<213>  Artificial Sequence

<220>
<223>  primer

<400>  4
gcgcatttaa atcgccacgc tgtgcctgac ga                                    32


<210>  5
<211>  1785
<212>  DNA
<213>  Artificial Sequence

<220>
<223>  Artificially mutated gene derived from Aeromonas caviae

<400>  5
atgagccaac catcttatgg cccgctgttc gaggccctgg cccactacaa tgacaagctg      60

ctggccatgg ccaaggccca gacagagcgc accgcccagg cgctgctgca gaccaatctg     120

gacgatctgg gccaggtgct ggagcagggc agccagcaac cctggcagct gatccaggcc     180

cagatgaact ggtggcagga tcagctcaag ctgatgcagc acaccctgct caaaagcgca     240

ggccagccga gcgagccggt gatcaccccg gagcgcagcg atcgccgctt caaggccgag     300

gcctggagcg aacaacccat ctatgactac ctcaagcagt cctacctgct caccgccagg     360

cacctgctgg cctcggtgga tgccctggag ggcgtccccc agaagagccg ggagcggctg     420

cgtttcttca cccgccagta cgtcagcgcc atggccccca gcaacttcct ggccaccaac     480

cccgagctgc tcaagctgac cctggagtcc ggcggccaga acctggtgcg cggactggcc     540

ctcttggccg aggatctgga gcgcagcgcc gatcagctca acatccgcct gaccgacgaa     600

tccgccttcg agctcgggcg ggatctggcc ctgaccccgg gccgggtggt gcagcgcacc     660

gagctctatg agctcattca gtacagcccg actaccgaga cggtgggcaa gacacctgtg     720

ctgatagtgc cgcccttcat caacaagtac tacatcatgg acatgcggcc ccagaactcc     780

ctggtcgcct ggctggtcgc ccagggccag acggtattca tgatctcctg gcgcaacccg     840

ggcgtggccc aggcccaaat cgatctcgac gactacgtgg tggatggcgt catcgccgcc     900

ctggacggcg tggaggcggc caccggcgag cgggaggtgc acggcatcgg ctactgcatc     960

ggcggcaccg ccctgtcgct cgccatgggc tggctggcgg cgcggcgcca gaagcagcgg    1020

gtgcgcaccg ccaccctgtt cactaccctg ctggacttct cccagcccgg ggagcttggc    1080

atcttcatcc acgagcccat catagcggcg ctcgaggcgc aaaatgaggc caagggcatc    1140

atggacgggc gccagctggc ggtctccttc agcctgctgc gggagaacag cctctactgg    1200

aactactaca tcgacagcta cctcaagggt cagagcccgg tggccttcga tctgctgcac    1260

tggaacagcg acagcaccaa tgtggcgggc aagacccaca acagcctgct gcgccgtctc    1320

tacctggaga accagctggt gaagggggag ctcaagatcc gcaacacccg catcgatctc    1380

ggcaaggtga agacccctgt gctgctggtg tcggcggtgg acgatcacat cgccctctgg    1440

cagggcacct ggcagggcat gaagctgttt ggcggggagc agcgcttcct cctggcggag    1500

tccggccaca tcgccggcat catcaacccg ccggccgcca acaagtacgg cttctggcac 1560

aacggggccg aggccgagag cccggagagc tggctggcag gggcgacgca ccagggcggc 1620

tcctggtggc ccgagatgat gggctttatc cagaaccgtg acgaagggtc agagcccgtc 1680

cccgcgcggg tcccggagga agggctggcc cccgcccccg gccactatgt caaggtgcgg 1740

ctcaaccccg tgtttgcctg cccaacagag gaggacgccg catga 1785

<210> 6
<211> 34
<212> DNA
<213> Artificial Sequence

<220>
<223> primer

<400> 6
gcgcgcattt aaatcatggc atctacgccg tcgg 34

<210> 7
<211> 20
<212> DNA
<213> Artificial Sequence

<220>
<223> primer

<400> 7
gccttttctg cctgggtcta 20

<210> 8
<211> 40
<212> DNA
<213> Artificial Sequence

<220>
<223> primer

<400> 8
tagacccagg cagaaaaggc gaaaacgccc gcgattgcgg 40

<210> 9
<211> 34
<212> DNA
<213> Artificial Sequence

<220>
<223> primer

<400> 9
gcgcgcattt aaatacgctg gcgcgtttcg tctg 34

<210> 10
<211> 32
<212> DNA
<213> Artificial Sequence

<220>
<223>  primer

<400>  10
aaatagattt aaatgggaca gcagcaggat tt                                    32


<210>  11
<211>  38
<212>  DNA
<213>  Artificial Sequence

<220>
<223>  primer

<400>  11
gctggcggct gccgggggct cggtccccgc tattctgg                              38


<210>  12
<211>  38
<212>  DNA
<213>  Artificial Sequence

<220>
<223>  primer

<400>  12
ccagaatagc ggggaccgag cccccggcag ccgccagc                              38


<210>  13
<211>  33
<212>  DNA
<213>  Artificial Sequence

<220>
<223>  primer

<400>  13
aaatagattt aaatacaaag gcaaaggggt agc                                   33


<210>  14
<211>  34
<212>  DNA
<213>  Artificial Sequence

<220>
<223>  primer

<400>  14
gcgcgcattt aaatgcaagc agttcggcgt ggcg                                  34


<210>  15
<211>  25
<212>  DNA
<213>  Artificial Sequence

<220>
<223>  primer

<400>  15
gcttgctctt cctattcagt caggg                                              25


<210>  16
<211>  43
<212>  DNA
<213>  Artificial Sequence

<220>
<223>  primer

<400>  16
gcagagagac aatcaaatca tgaagaccta cgagaacatc gcc                          43


<210>  17
<211>  33
<212>  DNA
<213>  Artificial Sequence

<220>
<223>  primer

<400>  17
gcgcgcattt aaattcaggg aaagcgccgc agg                                     33


<210>  18
<211>  45
<212>  DNA
<213>  Artificial Sequence

<220>
<223>  primer

<400>  18
ccctgactga ataggaagag caagccccgg gcaagtacct tgccg                        45


<210>  19
<211>  22
<212>  DNA
<213>  Artificial Sequence

<220>
<223>  primer

<400>  19
catgatttga ttgtctctct gc                                                 22


<210>  20
<211>  842
<212>  DNA
<213>  Cupriavidus necator

<400>  20
cccgggcaag taccttgccg acatctatgc gctggcgcgc acgcgcctgg cgcgcgccgg        60

ctgtaccgag gtctacggcg gcgacgcctg caccgtggcc gacgccggtc gcttctactc       120

ctatcggcgc gatggcgtga ccggccgcat ggccagcctg gtctggctgg cggactgagc 180

ccgccgctgc ctcactcgtc cttgcccctg gccgcctgcg cgcgctcggc ttcagccttg 240

cgtcggcggc ggccgggcgt gcccatgatg tagagcacca gcgccaccgg cgccatgcca 300

tacatcagga aggtggcaac gcctgccacc acgttgtgct cggtgatcgc catcatcagc 360

gccacgtaga gccagccaat ggccacgatg tacatcaaaa attcatcctt ctcgcctatg 420

ctctggggcc tcggcagatg cgagcgctgc ataccgtccg gtaggtcggg aagcgtgcag 480

tgccgaggcg gattcccgca ttgacagcgc gtgcgttgca aggcaacaat ggactcaaat 540

gtctcggaat cgctgacgat tcccaggttt ctccggcaag catagcgcat ggcgtctcca 600

tgcgagaatg tcgcgcttgc cggataaaag gggagccgct atcggaatgg acgcaagcca 660

cggccgcagc aggtgcggtc gagggcttcc agccagttcc agggcagatg tgccggcaga 720

ccctcccgct ttgggggagg cgcaagccgg gtccattcgg atagcatctc cccatgcaaa 780

gtgccggcca gggcaatgcc cggagccggt tcgaatagtg acggcagaga gacaatcaaa 840

tc 842

<210> 21
<211> 53
<212> DNA
<213> Artificial Sequence

<220>
<223> primer

<400> 21
gggcaattgc acgtgcagag agacaatcaa atcatgagcc aaccatctta tgg 53

<210> 22
<211> 70
<212> DNA
<213> Artificial Sequence

<220>
<223> primer

<400> 22
gcgcgcacta gtcggctgcc gactggttga accaggccgg caggtcaggc tcatgccttg 60

gctttgacgt 70

<210> 23
<211> 20
<212> DNA
<213> Artificial Sequence

<220>
<223> primer

<400> 23
gtgcaattgg cgcaacgcaa 20

<210>  24
<211>  23
<212>  DNA
<213>  Artificial Sequence

<220>
<223>  primer

<400>  24
gggcaattgt ttcctgtgtg aaa                                              23


<210>  25
<211>  147
<212>  DNA
<213>  Escherichia coli

<400>  25
gcgcaacgca attaatgtga gttagctcac tcattaggca ccccaggctt tacactttat      60

gcttccggct cgtatgttgt gtggaattgt gagcggataa caatttcaca caggaaacaa     120

ttgcacgtgc agagagacaa tcaaatc                                         147


<210>  26
<211>  29
<212>  DNA
<213>  Artificial Sequence

<220>
<223>  primer

<400>  26
gtgcaattgt gagtagtgag ccgcgtgga                                        29


<210>  27
<211>  20
<212>  DNA
<213>  Artificial Sequence

<220>
<223>  primer

<400>  27
gcccgtctcc agtggtgccg                                                  20


<210>  28
<211>  40
<212>  DNA
<213>  Artificial Sequence

<220>
<223>  primer

<400>  28
cggcaccact ggagacgggc atggcgaccg gcaaaggcgc                            40

<210>  29
<211>  670
<212>  DNA
<213>  Cupriavidus necator

<400>  29
tgagtagtga gccgcgtgga ttctgcgcca tgcggttgtt cccgaacggt cgttctaaaa      60

aatttagcac gaccgttcac aaaatttcga ttcgcggata atatagcttt tgacgggctg     120

gaaagatcat gaaggtgctc gacctgcgct gcgcgcatga ccatggtttc gagggctggt     180

ttgcctcgga agaagatgcg cagtcgcaaa tctcgcgtga cctcgtccaa tgcccggtct     240

gtggcgacca cgccgtgacg cggctgccca gcgcgccgcg cctgaacctg tcgggcgcga     300

ccgcgcgcga aggcagcgcc aggccggcgc agccggctgc cgcaccggag acactgcaag     360

cgctctatat gaaggcagtg aagcaggtgc tggcacagac cgaggatgtt ggcgatcgct     420

ttgccgaaga ggcaaggcgc atgcactatg acgaggcgcc ggaacgcggc attcgcggtt     480

cggcctcggc ggaggaggtg caggcgctgg ccgaagaggg catcgagact ttcccgctcg     540

tggtgccgga tgcgctgaag cagacggctc actgaattgc gtcttgtgcc cgtttgctgt     600

cggcgcttga cgtgcaggca gaacaaaccg ccggcggccc cacgccaagc cggcaccact     660

ggagacgggc                                                            670


<210>  30
<211>  32
<212>  DNA
<213>  Artificial Sequence

<220>
<223>  primer

<400>  30
gcgcgcgaat tccccgggca agtaccttgc cg                                    32

## Description

### Technical Field

The present invention relates to a polyester resin composition, and particularly relates to an polyester resin composition for allowing a biodegradable polyester resin, which is decomposed by action of microorganisms, to be used as various industrial materials, a molded article formed from such a resin composition, and a method for manufacturing such a molded article.

### Background Art

In recent years, biodegradable plastics have been actively developed to solve problems that plastic wastes cause a large burden to be imposed onto the global environment, examples of the problem including an effect onto ecological systems, the generation of harmful gases when the wastes are burned, and global warming based on a large quantity of burning calories thereof.

Particularly, carbon dioxide emitted when plant-derived biodegradable plastics are burned is originally present in the air. Thus, the plant-derived biodegradable plastics do not cause an increase in carbon dioxide quantity in the atmosphere. This matter is called carbon neutral. Importance is attached to the matter under the Kyoto Protocol, in which target values are set for carbon dioxide reduction. Thus, an active use of the plant-derived biodegradable plastics has been desired.

Recently, from the viewpoint of biodegradability and carbon neutral, aliphatic polyester resins have attracted attention as plant-derived plastics. Particularly, polyhydroxyalkanoate (hereinafter, sometimes referred to as PHA) resins have attracted attention. Among PHA resins, a poly(3-hydroxybutyrate) homopolymer resin, a poly(3-hydroxybutyrate-co-3-hydroxyvalerate) copolymer resin, a poly(3-hydroxybutyrate-co-3-hydroxyhexanoate) copolymer resin, a poly(3-hydroxybutyrate-co-4-hydroxybutyrate) copolymer resin, and polylactic acid have attracted attention.

However, such polyhydroxyalkanoate has a slow crystallization speed, and therefore when subjected to molding, it requires a long cooling time for solidification after heat-melting, which causes problems such as poor productivity and temporal change in mechanical properties (especially, toughness such as tensile elongation at break) due to secondary crystallization that occurs after molding.

Therefore, adding to a polyhydroxyalkanoate an inorganic material such as boron nitride, titanium oxide, talc, lamellar silicates, calcium carbonate, sodium chloride, or metal phosphates has heretofore been proposed to improve crystallization. However, addition of such an inorganic material has many harmful influences such that the obtained molded article is lowered in tensile elongation, the surface of the molded article is deteriorated in surface appearance, and when the molded article is made into a film, the transparency thereof is damaged, and therefore the effect is not sufficient.

As another method to improve crystallization of a polyhydroxyalkanoate without using such an inorganic material, a method of adding a polyhydroxybutyrate has been proposed (Patent Literatures 1 to 5).

However, in the method described in Patent Literature 1, a resin composition is produced by melt-kneading a polyhydroxyalkanoate and a polyhydroxybutyrate. When such melt-kneading is performed at a temperature equivalent to or higher than the melting point of the polyhydroxybutyrate in order to uniformly disperse the polyhydroxybutyrate in the polyhydroxyalkanoate, there is a problem that the thermal decomposition of the polyhydroxyalkanoate would progress in a short period of time. That is, in the method described in Patent Literature 1, it is difficult to satisfactorily disperse the polyhydroxybutyrate without advancing the deterioration of the polyhydroxyalkanoate due to heat, and the crystallization improving effect tends to be insufficient.

Further, Patent Literatures 2 to 5 describe a method of mixing a polyhydroxybutyrate adjusted in advance to small particle size and a polyhydroxyalkanoate. However, since there is a technical limitation in reducing the particle size of polyhydroxybutyrate, the crystallization improving effect is limited similarly as in Patent Literature 1, and thus there is still a room for improvement in this respect.

### Citation List

### Patent Literature

PTL 1: JP H08-510498 W
PTL 2: JP 2004-161802 A
PTL 3: JP 2005-162884 A
PTL 4: JP 2004-331757 A
PTL 5: JP 2004-331913 A

### Summary of Invention

### Technical Problem

An object of the present invention is to significantly improve the slow crystallization speed that is a disadvantage of biodegradable polyesters that are decomposed into water and carbon dioxide by action of microorganisms, especially polyhydroxyalkanoate, and to improve solidification properties in melt-molding to thereby enhance processing speed.

### Solution to Problem

The present inventors have found that a resin composition comprising a first polyhydroxyalkanoate, and a second polyhydroxyalkanoate having certain thermal properties solidifies quickly in molding and shows excellent productivity. The present invention has been completed based on these findings.

The present invention relates to a polyester resin composition comprising a first polyhydroxyalkanoate and a second polyhydroxyalkanoate, wherein a melting point of the second polyhydroxyalkanoate in the polyester resin composition is observed at a temperature lower than a melting point measured for the second polyhydroxyalkanoate alone.

When the resin composition is molded into a 100 µm-thick sheet, the number of foreign substances per 100 cm² of the sheet is preferably 20 or less.

Preferably, the melting point of the second polyhydroxyalkanoate in the polyester resin composition is observed at a temperature lower by 1 to 50°C than the melting point measured for the second polyhydroxyalkanoate alone.

Preferably, the first polyhydroxyalkanoate and the second polyhydroxyalkanoate are those that are simultaneously produced by one kind of microorganism.

The second polyhydroxyalkanoate is preferably poly-3-hydroxybutyrate.

The first polyhydroxyalkanoate is preferably at least one selected from the group consisting of poly(3-hydroxybutyrate-co-3-hydroxyhexanoate), poly(3-hydroxybutyrate-co-3-hydroxyvalerate), and poly(3-hydroxybutyrate-co-4-hydroxybutyrate).

The present invention also relates to a molded article comprising the resin composition.

In addition, the present invention also relates to a method for manufacturing the molded article, the method comprising processing the resin composition.

### Advantageous Effects of Invention

According to the present invention, the slow crystallization speed that is a disadvantage of polyhydroxyalkanoate can be significantly improved, and the solidification properties in the melt-molding can be improved to enhance the processing speed.

### Description of Embodiments

Hereinafter, the present invention will be described in more detail.

The polyester resin composition of the present invention includes at least a first polyhydroxyalkanoate and a second polyhydroxyalkanoate, and when the melting point of the polyester resin composition is measured, the melting point of the second polyhydroxyalkanoate contained in the polyester resin composition is characterized by being observed at a temperature lower than the melting point measured for the second polyhydroxyalkanoate alone. In contrast, in the case of measuring the melting point for a conventional polyester resin composition including two kinds of polyhydroxyalkanoates, the melting point of the second polyhydroxyalkanoate contained in the conventional polyester resin composition is observed at the same temperature with the melting point measured for the second polyhydroxyalkanoate alone. Since the polyester resin composition of the present invention has the above characteristics, the solidification in the melt-molding is faster as compared with the conventional polyester resin composition, so that an effect of excellent productivity is worked.

The cause that the melting point of the second polyhydroxyalkanoate in the resin composition of the present invention is observed at a low temperature is not clear, but such a cause is presumed to be derived from the dispersion state of the second polyhydroxyalkanoate in the resin composition of the present invention. In other words, the reason is considered to exist in that the second polyhydroxyalkanoate is dispersed at molecular level in the first polyhydroxyalkanoate, and the second polyhydroxyalkanoate has not grown in a crystal size enough to form a domain. Since melting point is generally correlated with crystal size, it is considered that the second polyhydroxyalkanoate has not grown into crystals of sufficient size, and is dispersed at molecular level. For this reason, it is presumed that the melting point of the second polyhydroxyalkanoate contained in the resin composition of the present invention is observed at a temperature lower than the melting point measured for the second polyhydroxyalkanoate alone.

The melting point in the present invention refers to a melting point as measured by a general differential scanning calorimetry (DSC) method. Specifically, the melting point refers to an endothermic peak measured at a temperature rising rate of 10°C/minute with use of a differential scanning calorimeter.

### [First Polyhydroxyalkanoate]

The first PHA used in the present invention is an aliphatic polyester containing a repeating unit represented by the formula (2):

[-CHR-CH₂-CO-O-]

wherein R is an alkyl group represented by CₙH₂ₙ₊₁, and n is an integer of 1 or more and 15 or less.

The first PHA can be produced, for example, by a microorganism such as Alcaligenes eutrophus AC32 strain (J. Bacteriol., 179, 4821 (1997)) which is obtained by introducing a PHA synthase gene derived from Aeromonas caviae into Alcaligenes eutrophus.

The first PHA used in the present invention is preferably at least one selected from PHAs produced from microorganisms.

The first PHA is preferably a resin containing 80 mol% or more of 3-hydroxybutyrate, more preferably a resin containing 85 mol% or more of 3-hydroxybutyrate, and is preferably a resin produced by a microorganism. Specific examples thereof include poly(3-hydroxybutyrate-co-3-hydroxypropionate), poly(3-hydroxybutyrate-co-3-hydroxyvalerate), poly(3-hydroxybutyrate-co-3-hydroxyvalerate-co-3-hydroxyhexanoate), poly(3-hydroxybutyrate-co-3-hydroxyhexanoate), poly(3-hydroxybutyrate-co-3-hydroxyheptanoate), poly(3-hydroxybutyrate-co-3-hydroxyoctanoate), poly(3-hydroxybutyrate-co-3-hydroxynonanoate), poly(3-hydroxybutyrate-co-3-hydroxydecanoate), poly(3-hydroxybutyrate-co-3-hydroxyundecanoate), poly(3-hydroxybutyrate-co-4-hydroxybutyrate), and the like. Among these, from the viewpoint of mold- processability and physical properties of the molded article, poly(3-hydroxybutyrate), poly(3-hydroxybutyrate-co-3-hydroxyvalerate), poly(3-hydroxybutyrate-co-3-hydroxyvalerate-co-3-hydroxyhexanoate), poly(3-hydroxybutyrate-co-3-hydroxyhexanoate), and poly(3-hydroxybutyrate-co-4-hydroxybutyrate) can be suitably used.

Further, since the first PHA is likely to obtain an effect of improving crystallization, either poly(3-hydroxybutyrate-co-3-hydroxyhexanoate) or poly(3-hydroxybutyrate-co-4-hydroxybutyrate), or a resin containing both is preferable.

From the viewpoint of mold- processability and quality of the molded article, the constituent ratio of 3-hydroxybutyrate (hereinafter, sometimes referred to as 3HB) to comonomer(s) copolymerized therewith (for example, 3-hydroxyvalerate (hereinafter, sometimes referred to as 3HV), 3-hydroxyhexanoate (hereinafter, sometimes referred to as 3HH), and 4-hydroxybutyrate (hereinafter, sometimes referred to as 4HB)) in the first PHA, that is, the ratio between monomers in the copolymer resin, is preferably 3-hydroxybutyrate/comonomer(s) =97/3 to 80/20 (mol%/mol%), more preferably 95/5 to 85/15 (mol%/mol%). If the comonomer(s) ratio is less than 3 mol%, the molding temperature and the pyrolysis temperature are close to each other, and therefore the composition may not be easily molded. If the comonomer(s) ratio exceeds 20 mol%, the PHA is slowly crystallized so that the PHA may be deteriorated in productivity. The comonomer(s) used may be one kind, and two or more comonomers may be used. Even when two or more comonomers are used, a preferred range of the ratio between the monomers (3-hydroxybutyrate/comonomer) in the resin is the same as defined above.

The ratio between respective monomers in the first PHA resin can be measured by gas chromatography in the following manner. Two milliliters of a mixed liquid of sulfuric acid/methanol (15/85 (weight ratio)) and 2 ml of chloroform are added to about 20 mg of dry PHA, and the resulting mixture is hermetically sealed and heated at 100°C for 140 minutes to obtain a methyl ester of a decomposition product of the PHA. After cooling, 1.5 g of sodium hydrogen carbonate is added thereto little by little for neutralization, and the resulting mixture is allowed to stand until the generation of carbon dioxide gas is stopped. Four milliliters of diisopropyl ether was added thereto, and the components are sufficiently mixed with each other. Thereafter, in the supernatant, the monomer unit composition of the PHA decomposition product is analyzed through capillary gas chromatography. In this way, the ratio between the respective monomers in the resin can be obtained.

A gas chromatograph used for the above measurement is "GC-17A" manufactured by Shimadzu Corporation. A capillary column used is "NEUTRA BOND-1" manufactured by GL Sciences Inc. (column length: 25 m, column internal diameter: 0.25 mm, and liquid film thickness: 0.4 µm). As a carrier gas, He is used. The pressure in an inlet of the column is set to 100 kPa, and the amount of an injected sample is 1 µL. Regarding temperature conditions, the sample temperature is raised from an initially starting temperature of 100 to 200°C at a rate of 8°C/minute, and further raised from 200 to 290°C at a rate of 30°C/minute.

The weight average molecular weight (hereinafter, sometimes referred to as Mw) of the first PHA is preferably 200000 to 2500000, more preferably 250000 to 2000000, even more preferably 300000 to 1000000. If the weight average molecular weight is less than 200000, the mechanical properties and the like may be poor. If the weight average molecular weight exceeds 2500000, the resin composition may not be easily molded.

For a method for measuring the above-described weight average molecular weight, a gel permeation chromatography ("Shodex GPC-101" manufactured by Showa Denko K.K.) is used. In its column, a polystyrene gel ("Shodex K-804" manufactured by Showa Denko K.K.) is used. Chloroform is used for its mobile phase. The molecular weight can be obtained as a molecular weight in terms of polystyrene. A calibration curve at this time is prepared, using polystyrene species having weight average molecular weights of 31400, 197000, 668000 and 1920000, respectively.

### [Second Polyhydroxyalkanoate]

The second polyhydroxyalkanoate is a polyhydroxyalkanoate which acts as a nucleating agent to improve the crystallization, namely the solidification of the first polyhydroxyalkanoate.

The second polyhydroxyalkanoate is a polyhydroxyalkanoate including a repeating unit represented by the formula (2) and having a structure different from the structure of the first polyhydroxyalkanoate.

The content of the second polyhydroxyalkanoate in the present invention is not particularly limited as long as it is less than the content of the first polyhydroxyalkanoate as a matrix. The content of the second polyhydroxyalkanoate is preferably 0.1 to 15 parts by weight per 100 parts by weight of the first polyhydroxyalkanoate. The above content is more preferably 0.3 to 10 parts by weight, even more preferably 0.5 to 8 parts by weight. If the content of the second polyhydroxyalkanoate is less than 0.1 part by weight, an effect of improving crystallization is inferior, and if the content of the second polyhydroxyalkanoate exceeds 15 parts by weight, the physical properties may be reduced such that the molded article is lowered in tensile elongation, the surface of the molded article is deteriorated in surface appearance, and when the molded article is made into a film, the transparency thereof is lost.

The second polyhydroxyalkanoate is a polyhydroxyalkanoate having a structure that is different from the structure of the first polyhydroxyalkanoate, and is preferably selected from the polyhydroxyalkanoates exemplified in the first polyhydroxyalkanoate, poly-3-hydroxybutyrates, and the like. For example, the second PHA is preferably a resin containing 80 mol% or more of 3-hydroxybutyrate, more preferably a resin containing 85 mol% or more of 3-hydroxybutyrate, and the second PHA is preferably one that is produced by a microorganism.

Among them, from the viewpoint of improving crystallization as well as dispersibility, poly-3-hydroxybutyrate can be particularly preferably used.

The poly-3-hydroxybutyrate is a resin containing 3-hydroxybutyrate as a main unit. Here, the "main unit" means that 3-hydroxybutyrate is present in an amount of 97.5 mol% or more, preferably 98 mol% or more, even more preferably 98.5 mol% or more, of the total monomer units constituting the resin.

The poly-3-hydroxybutyrate in the present invention is most preferably a homopolymer consisting of 3-hydroxybutyrate units (homopolymer), but is not limited thereto, and may be a resin obtained by copolymerizing a 3-hydroxybutyrate unit as a main unit and other hydroxyalkanoate units.

Examples of the other hydroxyalkanoate units include 3-hydroxyalkanoates such as 3-hydroxypropionate, 3-hydroxyhexanoate, 3-hydroxyvalerate, 3-hydroxycaproate, 3-hydroxyheptanoate, 3-hydroxyoctanoate, ω-fluoro-3-hydroxyheptanoate, ω-fluoro-3-hydroxynonanoate, and ω-chloro-3-hydroxyoctanoate; 2-hydroxyalkanoates such as glycolic acid, lactic acid, 2-hydroxybutyrate, 2-hydroxyisobutyrate, 2-hydroxy-2-methylbutyrate, 2-hydroxyvalerate, 2-hydroxycaproate, and 2-hydroxy-2-ethylbutyrate; 4-hydroxyalkanoates such as 4-hydroxybutyrate; 5-hydroxyalkanoates such as 5-hydroxyvalerate; and the like.

The weight average molecular weight of the second polyhydroxyalkanoate is not particularly limited as long as it can improve crystallization of the first polyhydroxyalkanoate. However, the weight average molecular weight of the second polyhydroxyalkanoate preferably falls within the same range as the weight average molecular weight of the first polyhydroxyalkanoate described above.

It should be noted that a poly-3-hydroxybutyrate-producing bacterium is Bacillus megaterium that is first discovered in 1925. Other natural microorganisms such as Cupriavidus necator (formerly classified as Alcaligenes eutrophus and Ralstonia eutropha) and Alcaligenes latus are known as the poly-3-hydroxybutyrate-producing microorganism. These microorganisms produce a poly-3-hydroxybutyrate by accumulating it in their cells.

### [Polyester Resin Composition]

When the melting point for the polyester resin composition of the present invention is measured, the melting point of the second polyhydroxyalkanoate is observed at a temperature lower than the melting point measured for the second polyhydroxyalkanoate alone.

The polyester resin composition of the present invention is not particularly limited as long as the melting point of the second polyhydroxyalkanoate in the composition is observed at a temperature lower than the melting point measured for the second polyhydroxyalkanoate alone. However, in view of the improvement of crystallization and excellent appearance of the molded article, it is preferred that the melting point of the second polyhydroxyalkanoate in the composition is observed at a temperature lower by preferably 1 °C to 50°C, more preferably 3°C to 45°C, even more preferably 5°C to 35°C, than the melting point measured for the second polyhydroxyalkanoate alone.

For example, in the case of poly 3-hydroxybutyrate having a melting point of 170°C when measured alone, the melting point of the poly-3-hydroxybutyrate measured in the polyester compositions of the present invention is preferably 120°C to 169°C, more preferably 125°C to 167°C, even more preferably 135°C to 165°C.

Preferably, a plurality of measurement samples are obtained in order to reduce a measurement error with respect to the melting point, the melting point of each of the samples was measured, and the average value thereof was adopted as a measurement value of the melting point.

The combination of the first polyhydroxyalkanoate and the second polyhydroxyalkanoate (first polyhydroxyalkanoate/second polyhydroxyalkanoate) is not particularly limited as long as such combination can improve crystallization of the first polyhydroxyalkanoate, but preferred are the combination of poly(3-hydroxybutyrate-co-3-hydroxyhexanoate)/poly-3-hydroxybutyrate, the combination of poly(3-hydroxybutyrate-co-3-hydroxyvalerate)/poly-3-hydroxybutyrate, and the combination of poly(3-hydroxybutyrate-co-4-hydroxybutyrate)/poly-3-hydroxybutyrate.

In the polyester resin composition of the present invention, since the second polyhydroxyalkanoate is well dispersed in the first polyhydroxyalkanoate and the melting point of the second polyhydroxyalkanoate is lowered, the second polyhydroxyalkanoate can be melted at a processing temperature of the first polyhydroxyalkanoate during the melt-molding of the polyester resin composition of the present invention, and yet it is possible to efficiently obtain an effect of improving the crystallization. Therefore, it is possible to reduce deterioration due to thermal decomposition during melt-molding, thereby making it possible to suppress the temporal change in mechanical properties after solidification.

Further, it is preferable that the second polyhydroxyalkanoate is finely dispersed in the polyester resin composition of the present invention to the extent that the molded article having a small number of foreign substances (foreign substances with large particle size) can be obtained. For example, the number of foreign substances in the polyester resin composition of the present invention is preferably 20 or less per 100 cm², more preferably 10 or less per 100 cm². Furthermore, from the viewpoint of obtaining a molded article having a thickness of less than 100 µm with less foreign substances, the number of foreign substances in the polyester resin composition is preferably 10 per 100 cm², more preferably 5 per 100 cm².

The number of foreign substances in the present invention means the number of foreign substances each with a size of 100 film or more, the substances being observed on the surface of the sheet with a thickness of 100 µm obtained by molding the polyester resin composition.

Further, in the polyester resin composition of the present invention, it is more preferred that there is no foreign substance with a size of 1 mm or more in a range of about 1,000 cm².

It is to be noted that the foreign substances in this application are derived from the resin components of large particle size due to insufficient mixing or dispersion, for example, unmelted resin, in the resin composition of the present invention.

Here, the processing temperature of the first polyhydroxyalkanoate refers to a temperature enabling the first polyhydroxyalkanoate to soften or flow to plasticization, including the melting point of the first polyhydroxyalkanoate.

The polyester resin composition according to the present invention may contain various additives in a range of not inhibiting the effects of the present invention. Examples of the additives include lubricants, crystal nucleating agents, plasticizers, hydrolysis suppressors, antioxidants, release agents, ultraviolet absorbers, colorants such as dyes and pigments, and inorganic fillers. These additives can be used according to the intended use of the polyester resin composition, and they are preferably biodegradable.

Moreover, the crystal nucleating agents as mentioned above refers to those that act as a nucleus at the time of crystallization of the first polyhydroxyalkanoate, excluding the second polyhydroxyalkanoate.

Other examples of the additives include inorganic fibers such as carbon fiber, and organic fibers such as human hair and wool. The other additives may be natural fibers such as bamboo fiber, pulp fiber, kenaf fiber, and a natural fiber of any other similar plant-alternate species, an annual herb plant in Hibiscus genus, Malvaceae family or an annual herb plant in Tiliaceae family. From the viewpoint of carbon dioxide reduction, a plant-derived natural fiber is preferred, and kenaf fiber is particularly preferred.

### [Method for Producing Polyester Resin Composition]

As a method for producing the polyester resin composition of the present invention, there can be preferably used a method of producing a first polyhydroxyalkanoate and a second polyhydroxyalkanoate simultaneously in one kind of microorganism, that is, a simultaneous production method thereof, from the viewpoint of being capable of satisfactorily dispersing the second polyhydroxyalkanoate while suppressing thermal deterioration without requiring a high-temperature process in the production.

Such microorganisms may be those having a gene encoding a PHA synthase for synthesizing a first PHA and a gene encoding a PHA synthase for synthesizing a second PHA. Such microorganisms have not been found so far in nature, but can be produced by introducing either a gene encoding a PHA synthase for synthesizing a first PHA or a gene encoding a PHA synthase for synthesizing a second PHA, or both of them into a microorganism as a host by using a gene recombination technique or the like. A known gene may be used as the gene to be introduced. Further, the microorganism of the present invention has preferably an "expression regulatory sequence" involved in expression at the upstream of a gene encoding a PHA synthase for synthesizing a first PHA and a gene encoding a PHA synthase for synthesizing a second PHA, respectively.

The polyester resin composition of the present invention can be produced by culturing the microorganism as described above to produce the first PHA and the second PHA simultaneously in the cells of the microorganism and collecting both PHAs from the microorganism.

The resin composition containing the first PHA and the second PHA simultaneously produced in this way from one kind of microorganism is different from a conventional resin composition produced by mixing the first PHA and the second PHA and can have characteristics that the melting point of the second polyhydroxyalkanoate is observed at a temperature lower than the melting point measured for the second polyhydroxyalkanoate alone.

The polyester resin composition of the present invention collected from the microorganism may be directly subjected to molding, but, such a resin composition maybe, after the addition of additives as needed, melt kneaded, pelletized, and then subjected to molding.

It is possible to form pellets by mechanically kneading the polyester resin composition while heating at a suitable temperature, utilizing a known apparatus such as a Banbury mixer, a roll mill, a kneader, or a single or multi screw extruder. The temperature at the time of kneading, which may be adjusted depending on the melting temperature of the resin used, is preferably about 185°C as the upper limit in order to obtain the effect of the present invention to improve crystallization of the polyhydroxyalkanoate. Thus, the kneading temperature is, for example, about 100 to 185°C, preferably 170°C or less.

### [Molded article Containing Polyester Resin Composition]

A method of manufacturing a molded article containing the polyester resin composition of the present invention will be illustrated below.

The polyester resin composition of the present invention or its pellets are sufficiently dried at 40 to 80°C to remove moisture, and then can be subjected to molding by a known molding method to obtain any molded article. Examples of the molding method include film forming, sheet forming, injection molding, blow molding, fiber spinning, extrusion foaming, bead foaming, and the like.

Examples of the method for manufacturing a film molded article include T-die extrusion forming, calendar forming, roll molding, and inflation forming. However, the film forming method is not limited thereto. The temperature at which film forming is performed is preferably 140 to 190°C, more preferably 140 to 170°C. Further, a film obtained from the polyester resin composition according to the present invention can be subjected to thermoforming under heating, vacuum forming, or press forming.

The method for manufacturing an injection-molded article may be, for example, an injection molding method generally adopted to mold a thermoplastic resin, or an injection molding method such as a gas assist molding method or an injection compression molding method. In accordance with the purpose, the following methods other than the above-described methods may be adopted: in-mold molding method, gas pressing method, two-color molding method, sandwiching molding method, and push-pull molding method, and SCORIM. However, the injection molding method is not limited to thereto. At the time of the injection molding, the molding temperature is preferably from 140 to 190°C, and the mold temperature is preferably from 20 to 80°C, more preferably 30 to 70°C.

The molded article according to the present invention can be appropriately used in the fields of agriculture, fishery, forestry, gardening, medicine, sanitary goods, food industry, clothing, non-clothing, packaging, cars, building materials, or other areas.

### Examples

Hereinafter, the present invention will be described specifically by way of examples, but the technical scope of the present invention is not limited by these examples.

### <Example 1> Preparation of PHA Using KNK005 REP-phaJ4b/ΔphaZ1,2,6/pCUP2-Plac-phaC_{Re} Strain

### (Product of 2 wt% of PHB)

First, a plasmid for gene substitution was prepared for the purpose of disruption of phaZ6 gene. PCR was performed using the genomic DNA of C. necator H16 strain as a template and DNAs as set forth in SEQ ID NO: 1 and SEQ ID NO: 2 as primers. KOD-plus (manufactured by Toyobo Co., Ltd.) was used as the polymerase. Similarly, PCR was performed using DNAs as set forth in SEQ ID NO: 3 and SEQ ID NO: 4 as primers. Furthermore, using as a template two DNA fragments obtained in the above PCR, PCR was performed with DNAs as set forth in SEQ ID NO: 1 and SEQ ID NO: 4 as primers, and the resulting DNA fragment was digested with a restriction enzyme SmiI. This DNA fragment was joined, using a DNA ligase (Ligation High, manufactured by Toyobo Co., Ltd.), to a DNA fragment which was obtained by digesting a vector pNS2X-sacB as described in JP 2007-259708 A with SmiI, so that a gene disruption plasmid pNS2X-phaZ6 (-+) was prepared which had DNA sequences upstream and downstream of the phaZ6 structural gene.

Then, a strain for gene disruption was prepared. The gene disruption plasmid pNS2X-phaZ6 (-+) was introduced into E. coli strain S 17-1 (ATCC 47055), which was then mix-cultured with a KNK005 strain (see US 7384766) on a nutrient agar medium (manufactured by DIFCO) to cause conjugal transfer. The KNK005 strain is a strain having a gene encoding PHA synthase as set forth in SEQ ID NO: 5 and its host is C. necator H16 strain.

From the strains after the conjugal transfer, a strain grown on Simmons agar medium (sodium citrate 2 g/L, sodium chloride 5 g/L, magnesium sulfate heptahydrate 0.2 g/L, ammonium dihydrogen phosphate 1 g/L, dipotassium hydrogen phosphate 1 g/L, agar 15 g/L, pH 6.8) containing 250 mg/L of kanamycin sulfate was selected to obtain a strain wherein the plasmid is incorporated onto the chromosome of the KNK005. This strain was cultured on nutrient broth medium (manufactured by DIFCO) through two generations, and strains grown on nutrient agar medium containing 15% sucrose were selected. Those in which the full length from the start codon of the phaZ6 gene to the stop codon was deleted were selected from the obtained strains by PCR, and one strain among these was named KNK005 ΔphaZ6 strain. The KNK005 ΔphaZ6 strain is a strain in which the full length of the phaZ6 gene on the chromosome is deleted and a gene encoding the PHA synthase as set forth in SEQ ID NO: 5 on the chromosome is contained.

Next, a plasmid for gene substitution was prepared for the purpose of disruption of phaZ1 gene. PCR was performed using the genomic DNA of C. necator H16 strain as a template and DNAs as set forth in SEQ ID NO: 6 and SEQ ID NO: 7 as primers. KOD-plus was used as the polymerase. Similarly, PCR was performed using DNAs as set forth in SEQ ID NO: 8 and SEQ ID NO: 9 as primers. Furthermore, using as a template two DNA fragments obtained in the above PCR, PCR was performed with DNAs as set forth in SEQ ID NO: 6 and SEQ ID NO: 9 as primers, and the resulting DNA fragment was digested with a restriction enzyme SmiI. This DNA fragment was joined, using a DNA ligase, to a DNA fragment which was obtained by digesting pNS2X-sacB with Smil, so that a plasmid pNS2X-phaZ1 (-+) for gene disruption was prepared which had DNA sequences upstream and downstream of the phaZ1 structural gene.

In the same manner as in the disruption of the phaZ6 gene, disruption of the phaZ1 gene was performed using the KNK005 ΔphaZ6 strain, serving as a parent strain, and pNS2X-phaZ1(-+). The resulting strain was named KNK005 ΔphaZ1,6 strain. The KNK005 ΔphaZ1,6 strain is a strain in which the full length of the phaZ1 gene and the phaZ6 gene on the chromosome is deleted, and a gene encoding the PHA synthase as set forth in SEQ ID NO: 5 on the chromosome is contained.

Next, a plasmid for gene substitution was prepared for the purpose of disruption of phaZ2 gene. PCR was performed using the genomic DNA of C. necator H16 strain as a template and DNAs as set forth in SEQ ID NO: 10 and SEQ ID NO: 11 as primers. KOD-plus was used as the polymerase. Similarly, PCR was performed using DNAs as set forth in SEQ ID NO: 12 and SEQ ID NO: 13 as primers. Furthermore, using as a template two DNA fragments obtained in the PCR described above, PCR was performed with DNAs as set forth in SEQ ID NO: 10 and SEQ ID NO: 13 as primers, and the resulting DNA fragment was digested with a restriction enzyme Smil. This DNA fragment was joined, using a DNA ligase, to a DNA fragment which was obtained by digesting pNS2X-sacB with SmiI, so that a plasmid pNS2X-phaZ2 (-+) for gene disruption was prepared which had DNA sequences upstream and downstream of the phaZ2 structural gene.

In the same manner as in the disruption of the phaZ6 gene, disruption of the phaZ2 gene was performed using the KNK005 ΔphaZ1,6 strain, serving as a parent strain, and pNS2X-phaZ2(-+). The resulting strain was named KNK005 ΔphaZ1,2,6 strain. The KNK005 ΔphaZ1,2,6 strain is a strain in which the full length of the phaZ1 gene and the phaZ6 gene on the chromosome is deleted; the length from the 16th codon of the phaZ2 gene to the stop codon is deleted; and a gene encoding the PHA synthase as set forth in SEQ ID NO: 5 on the chromosome is contained.

Further, for the purpose of inserting an expression regulatory sequence into upstream of phaJ4b gene on the chromosome, a plasmid for inserting an expression regulatory sequence was prepared. PCR was performed using the genomic DNA of C. necator H16 strain as a template and DNAs as set forth in SEQ ID NO: 14 and SEQ ID NO: 15 as primers. KOD-plus was used as the polymerase. Similarly, PCR was performed using DNAs as set forth in SEQ ID NO: 16 and SEQ ID NO: 17 as primers. Furthermore, PCR was performed similarly with DNAs as set forth in SEQ ID NO: 18 and SEQ ID NO: 19 as primers. Using as templates three DNA fragments obtained in the PCR described above, PCR was performed with DNAs as set forth in SEQ ID NO: 14 and SEQ ID NO: 17 as primers, and the resulting DNA fragment was digested with a restriction enzyme Smil. This DNA fragment was joined, using a DNA ligase, to a DNA fragment which was obtained by digesting pNS2X-sacB with Smil, so that a plasmid pNS2X-sacB + phaJ4bU-REP-phaJ4b for DNA insertion was prepared which had a DNA sequence upstream of the structural gene sequence of the phaJ4b, an expression regulatory sequence as set forth in SEQ ID NO: 20 composed of the phaC1 promoter and the phaC1SD sequence, and the structural gene sequence of the phaJ4b.

In the same manner as the above-mentioned gene disruption, an expression regulatory sequence was inserted into the upstream of the phaJ4b gene using the KNK005 ΔphaZ1,2,6 strain serving as a parent strain and the pNS2X-sacB + phaJ4bU-REP-phaJ4b. The resulting strain was named KNK005 REP-phaJ4b/ΔphaZ1,2,6 strain. The KNK005 REP-phaJ4b/ΔphaZ1,2,6 strain is a strain in which the full length of the phaZ1 gene and the phaZ6 gene on the chromosome is deleted; the length from the 16th codon of the phaZ2 gene to the stop codon is deleted; an expression regulatory sequence as set forth in SEQ ID NO: 20 composed of the phaC1 promoter and the phaC1SD sequence are inserted immediately upstream of the phaJ4b gene; and a gene encoding the PHA synthase as set forth in SEQ ID NO: 5 on the chromosome is contained.

Then a plasmid pCUP2-Plac-phaC_{Re} for PHB production was prepared for the purpose of simultaneously producing PHBH and PHB by introducing into the KNK005 REP-phaJ4b/ΔphaZ1,2,6 strain.

First, PCR was performed using the genomic DNA of C. necator H16 strain as a template and DNAs as set forth in SEQ ID NO: 21 and SEQ ID NO: 22 as primers, and the resulting DNA fragment was digested with MunI and SpeI. KOD-plus was used as the polymerase. This DNA fragment was joined, using a DNA ligase, to a DNA fragment which was obtained by digesting a pCUP2 vector with MunI and SpeI, so that a plasmid pCUP2-SD-phaC_{Re} for PHB production was prepared which had an expression regulatory sequence composed of phaC1SD sequence, and a phaC_{Re} structural gene sequence.

Then PCR was performed using pCR(R)2.1-TOPO(R) (manufactured by Invitrogen) as a template and DNAs as set forth in SEQ ID NO: 23 and SEQ ID NO: 24 as primers, and the resulting DNA fragment was digested with MunI. KOD-plus was used as the polymerase. This DNA fragment was joined, using a DNA ligase, to a DNA fragment which was obtained by digesting pCUP2-SD-phaC_{Re} with MunI, so that a plasmid pCUP2-Plac-phaC_{Re} for PHB production was prepared which had an expression regulatory sequence as set forth in SEQ ID NO 25 composed of the lac promoter and phaC1SD sequence, and a PhaC_{Re} structural gene sequence.

Then, the pCUP2-Plac-phaC_{Re} was introduced to KNK005 REP-phaJ4b/ΔphaZ1,2,6 strain. The KNK005 REP-phaJ4b/ΔphaZ1,2,6 strain was cultured overnight in nutrient broth medium. The resulting culture solution (0.5 ml) was inoculated into nutrient broth medium (100 ml), and incubated at 30°C for 3 hours. The obtained culture solution was quickly cooled on ice, and the cells were collected, washed well with ice-cold distilled water, and suspended in 2 ml of distilled water. The cells were mixed with the plasmid solution, injected into the cuvette, and subjected to electroporation. The electroporation was performed under conditions of a voltage of 1.5 kV, a resistance of 800 Ω, and a current of 25 µF, using MicroPulser electroporator (manufactured by Bio-Rad). After the electroporation, the cell solution was collected, 5 ml of nutrient broth medium was added thereto, and the cells were cultured at 30°C for 3 hours. The resulting culture solution was applied to a nutrient agar medium containing 100 mg/l of kanamycin sulfate, and cultured for 3 days at 30°C to obtain a strain into which the plasmid had been introduced, from the resulting colonies. The resulting strain was named KNK005 REP-phaJ4b/ΔphaZ1,2,6/pCUP2-Plac-phaC_{Re} strain.

### (Culture)

The KNK005 REP-phaJ4b/ΔphaZ1,2,6/pCUP2-Plac-phaC_{Re} strain was cultured in the following manner.

The composition of a seed medium was as follows: 1% (w/v) Meat-extract, 1% (w/v) Bacto-Tryptone, 0.2% (w/v) Yeast-extract, 0.9% (w/v) Na₂PO₄•12H₂O, and 0.15% (w/v) KH₂PO₄ (pH 6.8).

The composition of a preculture medium was as follows: 1.1% (w/v) Na₂PO₄•12H₂O, 0.19% (w/v) KH₂PO₄, 1.29% (w/v) (NH₄)₂SO₄, 0.1% (w/v) MgSO₄•7H₂O, 0.5% (v/v) trace metal salt solution (obtained by dissolving 1.6% (w/v) FeCl₂•6H₂O, 1% (w/v) CaCl₂•2H₂O, 0.02% (w/v) CoCl₂•6H₂O, 0.016% (w/v) CuSO₄•5H₂O, and 0.012% (w/v) NiCl₂•6H₂O in 0.1N hydrochloric acid), and 5 x 10⁻⁶% (w/v) kanamycin. As a carbon source, palm double olein oil was used at a concentration of 2.5% (w/v).

The composition of a PHA production medium was as follows: 0.578% (w/v) Na₂PO₄•12H₂O, 0.101% (w/v) KH₂PO₄, 0.437% (w/v) (NH₄)₂SO₄, 0.15% (w/v) MgSO₄•7H₂O, 0.75% (v/v) trace metal salt solution (obtained by dissolving 1.6% (w/v) FeCl₂•6H₂O, 1% (w/v) CaCl₂•2H₂O, 0.02% (w/v) CoCl₂•6H₂O, 0.016% (w/v) CuSO₄•5H₂O, and 0.012% (w/v) NiCl₂•6H₂O in 0.1N hydrochloric acid). As a carbon source, an emulsion obtained by emulsifying palm fatty acid distillate (PFAD; available from MALAYSIAN BIOTECHNOLOGY CORPORATION SDN BDH. Free fatty acid content 95.0%; fatty acid composition: C12:0 0.2%, C14:01.2%, C16:047.6%, C16:10.3%, C18:1 35.7%, C18:2 9.7%, C18:3 0.4%, C20:0 0.4%; the melting point of 43.8°C) according to the following procedure was used.

PFAD (550 g) and water (450 g) were weighed, and each was heated to 60°C. After that, 4.7 g of Na₂PO₄•12H₂O and 2.75 g of sodium caseinate were dissolved in water. After dissolution, the solution was mixed with PFAD, and the mixture was pre-emulsified at 2500 rpm using a homo-mixer (LABORATORY MIXER EMULSIFIER, manufactured by SILVERSON). In addition, this preliminary emulsion was subjected to an emulsification operation at a pressure of 10 barr, using a highpressure homogenizer (PAND2K type, manufactured by NIRO SOAVI), to thereby obtain an emulsion.

A glycerol stock (50 µl) of each strain was inoculated into the seed culture medium (10 ml) and cultured for 24 hours, and the culture was inoculated at 1.0% (v/v) into a 3Ljar fermenter (MDL-300 type, manufactured by B. E. Marubishi, Co., Ltd.) containing 1.8 L of the preculture medium. The operation conditions were as follows: culture temperature 30°C, stirring rate 500 rpm, and aeration amount 1.8 L/minute. The culture was performed for 28 hours while controlling the pH between 6.7 and 6.8. For the pH control, a 7% aqueous solution of ammonium hydroxide was used.

The PHA production culture was performed by inoculating the preculture seed at 25% (v/v) into a 10 L jar fermenter (MDL-1000 type, manufactured by B. E. Marubishi, Co., Ltd.) containing 2 L of the PHA production medium. The operation conditions were as follows: culture temperature 32°C, stirring rate 450 rpm, aeration amount 3.0 L/minute, and the pH controlled between 6.7 and 6.8. For the pH control, a 7% aqueous solution of ammonium hydroxide was used. The culture was performed for about 45 to 54 hours.

### (Purification)

During the culture and at the end of culture, the culture broth was sampled and centrifuged to collect the cells. The cells were washed with ethanol and dried under vacuum to obtain the dry cells.

To 1 g of the obtained dry cells was added 100 ml of chloroform, and the mixture was stirred at room temperature through whole day and night to extract PHA in the cells. After filtering the cell residues, the filtrate was concentrated to a total volume of 30 ml using an evaporator, gradually added with 90 ml of hexane, and stirred gently for 1 hour. The precipitated PHA was filtered and dried under vacuum at 50°C for 3 hours, to thereby obtain a purified PHA.

The obtained resin composition containing the PHBH and PHB was referred to as composition 1.

### <Example 2> Preparation of PHA Using KNK005 REP-phaJ4b/ΔphaZ1,2,6/pCUP2-PJ4a-phaC_{Re} Strain

### (Product of 3.5 wt% of PHB)

A plasmid pCUP2-PJ4a-phaC_{Re} for PHB production was prepared for the purpose of simultaneously producing PHBH and PHB by introducing into KNK005 REP-phaJ4b/ΔphaZ1,2,6 strain.

First, PCR was performed using the genomic DNA of C. necator H16 strain as a template and DNAs as set forth in SEQ ID NO: 26 and SEQ ID NO: 27 as primers. Similarly, PCR was performed using DNAs as set forth in SEQ ID NO: 22 and SEQ ID NO: 28 as primers. Using as a template two kinds of DNA fragments obtained in the PCR described above, PCR was performed with DNAs as set forth in SEQ ID NO: 22 and SEQ ID NO: 26 as primers, and the resulting DNA fragment was digested with MunI and Spel. KOD-plus was used as the polymerase. This DNA fragment was joined, using a DNA ligase, to a DNA fragment which was obtained by digesting a pCUP2 vector with MunI and SpeI, so that a plasmid pCUP2-PJ4a-phaC_{Re} for PHB production was prepared which had an expression regulatory sequence PJ4a as set forth in SEQ ID NO: 29 and a phaC_{Re} structural gene sequence.

In the same manner as in Example 1, the pCUP2-PJ4a-phaC_{Re} was introduced into the KNK005 REP-phaJ4b/ΔphaZ1,2,6 strain. The resulting strain was named as KNK005 REP-phaJ4b/ΔphaZ1 ,2,6/pCUP2-PJ4a-phaC_{Re} strain.

Using KNK005 REP-phaJ4b/ΔphaZ1,2,6/pCUP2-PJ4a-phaC_{Re} strain, a purified PHA was obtained in the same manner as in Example 1. The obtained resin composition containing the PHBH and PHB was referred to as composition 2.

### <Example 3> Preparation of PHA Using KNK005 REP-phaJ4b/ΔphaZ1,2,6/pCUP2-PJ4a-phaC_{Re} Strain

### (Product of 6.0 wt% of PHB)

Using KNK005 REP-phaJ4b/ΔphaZ1,2,6/pCUP2-PJ4a-phaC_{Re} strain, a purified PHA was obtained in the same manner as in Example 1, except that palm double olein oil was used as a carbon source during culture instead of the emulsified PFAD. The obtained resin composition containing the PHBH and PHB was referred to as composition 3.

### <Example 4> Preparation of PHA Using KNK005 REP-phaJ4b/ΔphaZ1,2,6/pCUP2-REP-phaC_{Re} Strain

### (Product of 7.9 wt% of PHB)

A plasmid pCUP2-REP-phaC_{Re} for PHB production was prepared for the purpose of simultaneously producing PHBH and PHB by introducing into KNK005 REP-phaJ4b/ΔphaZ1,2,6 strain.

First, PCR was performed using the genomic DNA of C. necator H16 strain as a template and DNAs as set forth in SEQ ID NO: 22 and SEQ ID NO: 30 as primers, and the resulting DNA fragment was digested with EcoRI and Spel. KOD-plus was used as the polymerase. This DNA fragment was joined, using a DNA ligase, to a DNA fragment which was obtained by digesting a pCUP2 vector described in JP 2007-259708 A with MunI and SpeI, so that a plasmid pCUP2-REP-phaC_{Re} for PHB production was prepared which had an expression regulatory sequence as set forth in SEQ ID NO: 20 composed of the phaC1 promoter and phaC1SD sequence, and a phaC_{Re} structural gene sequence.

In the same manner as in Example 1, the pCUP2-REP-phaC_{Re} was introduced into KNK005 REP-phaJ4b/ΔphaZ1,2,6 strain. The resulting strain was designated as KNK005 REP-phaJ4b/ΔphaZ1,2,6/pCUP2-REP-phaC_{Re} strain.

Using the KNK005 REP-phaJ4b/ΔphaZ1,2,6/pCUP2-REP-phaC_{Re} strain, a purified PHA was obtained in the same manner as in Example 1. The obtained resin composition containing the PHBH and PHB was referred to as composition 4.

### <Comparative Example 1> (Production of PHBH)

KNK-631 strain (see WO 2009/145164) was used for the culture production of PHBH. Such culture was performed in the same manner as in Example 1, except that palm double olein oil was used as a carbon source instead of the emulsified PFAD. The culture was performed for 60 to 70 hours.

PHBH from the culture solution was purified in the same manner as in Example 1. The obtained PHBH was referred to as composition 5.

### <Comparative Examples 2 and 3> (Melt-Kneading of PHBH and PHB)

The PHBH obtained in Comparative Example 1 and the PHB obtained in the following Production Example 1 were dry-blended at the composition ratio shown in Table 1, and melt-kneaded using a twin screw extruder TEM26SS of Toshiba Machine Co., Ltd., at a cylinder setting temperature of 120 to 140°C and a screw rotation speed of 100 rpm to prepare a resin composition. The obtained resin compositions were referred to as composition 6 and composition 7, respectively.

### <Production Example 1> (Production of PHB)

Cupriavidus necator H16 (ATCC 17699) strain was used in the culture production of PHB. The culture was performed in the same manner as in Example 1. The culture was performed for 45 to 54 hours.

PHB from culture solution was purified in the same manner as in Example 1.

Each composition obtained above was subjected to the following measurement.

### <Fractionation of Each Resin Component from Composition>

About 1000 mg of the resin composition was dissolved in 100 ml of chloroform at about 50°C. Thereafter, 266.7 ml of hexane was added thereto, and the mixture was stirred for 10 minutes to obtain a precipitated component. The resulting precipitated component was fractionated as a first precipitated fraction by suction filtration. Further, 130 ml of hexane was added thereto, and the mixture was stirred for 5 minutes, and allowed to stand for 15 minutes to obtain a precipitate. The resulting precipitate was suction-filtered as a second precipitated fraction. Each precipitate was dried and weighed, and a first precipitated fraction weight ratio to the total amount of the first precipitated fraction and the second precipitated fraction was shown in Table 1 as the ratio of PHB.

### <Composition Analysis of First Precipitated Fraction and Second Precipitated Fraction>

Composition analysis was determined by gas chromatography. After each precipitated fraction was dried at 80°C for 4 hours, 2 ml of sulfuric acid-methanol mixture (15:85) and 2 ml of chloroform were added to 20 mg of each precipitated fraction, and the mixture was sealed and heated at 100°C for 140 minutes to give a methyl ester. After cooling, this methyl ester was neutralized by gradually adding 1.5 g of sodium bicarbonate, and the mixture was allowed to stand until the generation of carbon dioxide was stopped. After mixing well with the addition of 4 ml of diisopropyl ether, the mixture was centrifuged, and the monomer unit composition in the supernatant was analyzed by capillary gas chromatography. A gas chromatograph used was GC-17A manufactured by Shimadzu Corporation. A capillary column used was NEUTRA BOND-1 manufactured by GL Sciences Inc. (column length: 25 m, column internal diameter: 0.25 mm, and liquid film thickness: 0.4 µm). As a carrier gas, He was used. The pressure in an inlet of the column was set to 100 kPa, and the amount of an injected sample was 1 µL. Regarding temperature conditions, the sample temperature was raised from an initially starting temperature of 100°C to 200°C at a rate of 8°C minute, and further raised from 200 to 290°C at a rate of 30°C/minute.

As a result of analysis under the above conditions, 3-hydroxyhexanoate was not detected from the first precipitated fraction, and the first precipitated fraction was found to be a poly(3-hydroxybutyrate) homopolymer (also herein referred to as PHB). Detection of 3-hydroxyhexanoate from the second precipitated fraction was confirmed, and the second precipitated fraction was found to be poly(3-hydroxybutyrate-co-3-hydroxyhexanoate) (also herein referred to as PHBH). The composition ratio of 3-hydroxyhexanoate (3HH) in the second precipitated fraction PHBH is shown in Table 1.

### <Melting Point Measurement of PHBH Alone or PHB Alone>

The melting point was measured using a differential scanning calorimeter (DSC7020) manufactured by Hitachi High-Tec Science Corporation. As a sample, about 5 mg of PHBH or PHB fractionated as above was accurately weighed, and an endothermic peak obtained at a temperature-rising rate of 10°C/minute was taken as the melting point. The average value of three times measurements was taken as a melting point and is shown in Table 1.

### <Melting Point Measurement of Composition>

The measurement of the melting point was performed using a differential scanning calorimeter (DSC7020) manufactured by Hitachi High-Tech Science Corporation. As a sample, about 5 mg of any one of the compositions 1 to 7 was accurately weighed, and an endothermic peak obtained at a temperature-rising rate of 10°C/min was taken as the melting point. The endothermic peak of the low-temperature side was taken as the melting point of PHBH, and the endothermic peak of the high-temperature side was taken as the melting point of PHB. The average value of three times measurements was taken as the melting point and is shown in Table 1.

### <Solidification Test>

The solidification test was performed as follows. First, any one of the compositions 1 to 7 was dried at 80°C for 5 hours. Then, using a small kneading machine Xplore series MC5 manufactured by DSM, Inc., each composition was melt-kneaded at a setting temperature of 170°C and a screw rotation speed of 100 rpm for a kneading time of 1 minute, and the molten resin was poured into a warm bath of about 50°C to measure the time required for solidification. The faster the crystallization is, the shorter the time required for solidification is. That is, when the number is small in the solidification test, it means that solidification properties are excellent. The measurement was repeated 5 times with the same procedure and the average value was shown in Table 1.

### <Pellet Productivity>

The pellet productivity was evaluated as follows. By using a twin screw extruder TEM26SS manufactured by Toshiba Machine Co., Ltd., each composition was melt-kneaded at a cylinder setting temperature of 120 to 140°C, and the molten resin was discharged from a strand die having a diameter of 4 mm and three holes, and passed through a 1.5 m-long warm bath filled with warm water set to 60°C, to thereby cause crystallization, and solidification. The obtained solid was cut into pellets by a pelletizer.

In order to raise the resin discharge rate to increase the pellet productivity, it is necessary to raise the screw rotation speed of the extruder so that the linear speed of the strand should be increased. An increase of the screw rotation speed increases the resin temperature due to shear heat generation, and the residence time of the resin in the warm bath becomes shorter as the linear speed is increased. When the resin temperature is increased, crystallization of the resin becomes difficult. Also, when the residence time of the resin in the warm bath is shortened, the resin will remain softened without complete crystallization. That is, when the resin temperature is increased and the residence time in the warm bath becomes shorter, cutting of the resin with a pelletizer becomes impossible.

The largest strand linear speed for permitting pelletization was defined as the pellet productivity. The pellet productivity becomes more excellent as the linear speed value becomes higher. The results are shown in Table 1.

### <Sheet Productivity in T-Die Forming>

The sheet productivity was evaluated as follows. Each composition was molded into a 100 mm-wide sheet using a T-die sheet formimg machine (Laboplastomill, manufactured by Toyo Seiki Seisakusho Co., Ltd.) under conditions where a die lip thickness was 250 µm, a die lip width was 150 mm, a cylinder setting temperature was 120 to 140°C, a die setting temperature was 150 to 160°C, and a cooling roll setting temperature was 60°C. The molten resin extruded through the T-die as a sheet is crystallized and solidified upon being brought into contact with a cooling roll, and is therefore formed into a 100 µm-thick sheet. When the resin is sufficiently crystallized and solidified, the formed sheet is released from the cooling roll and rolled up. However, when the linear speed of the sheet is increased, the contact time between the sheet and the cooling roll is shortened. As a result, the resin is not crystallized and is therefore not sufficiently solidified, so that the sheet cannot be released from the cooling roll. The maximum linear speed of the sheet at which the sheet could be released from the cooling roll was defined as sheet productivity. A higher linear speed value means better sheet productivity. The results are shown in Table 1.

### <Number of Foreign Substances in Sheet Obtained by T-Die Forming>

The number of foreign substances was evaluated as follows. The sheet with a thickness of about 100 µm obtained by T-die forming as described above was cut out into a piece of approximately 100 mm square, and the piece was observed using a magnifying glass of 20 times magnifications with scale, and the number of foreign substances (unmelted resin) with a size of 100 µm or more was counted. The value in terms of the number of foreign substances present per 100 cm² of area is shown in Table 1.

**[Table 1]**

| | | | Example 1 | Example 2 | Example 3 | Example 4 | Comparative Example 1 | Comparative Example 2 | Comparative Example 3 |
|---|---|---|---|---|---|---|---|---|---|
| | | | Composition 1 | Composition 2 | Composition 3 | Composition 4 | Composition 5 | Composition 6 | Composition 7 |
| 3HH ratio ofPHBH (first PHA) | | mol% | 10.3 | 11.9 | 10.9 | 10.8 | - | - | - |
| Melting point ofPHBH (first PHA) alone | | °C | 109 | 101 | 105 | 106 | - | - | - |
| Ratio ofPHB (second PHA) | | wt% | 2.6 | 3.8 | 5.0 | 8.7 | - | - | - |
| Melting point ofPHB (second PHA) alone | | °C | 174 | 174 | 173 | 173 | - | - | - |
| Blending ratio ofPHBH (note 3) | | wt% | - | - | - | - | 100 | 98 | 92 |
| Blending ratio ofPHB (note 4) | | wt% | - | - | - | - | 0 | 2 | 8 |
| Melting point observed in each composition | Melting point ofPHBH (first PHA) | °C | 110 | 100 | 107 | 108 | 107 | 108 | 109 |
| | Melting point ofPHB (second PHA) | °C | 154 | 154 | 164 | 156 | - | 174 | 174 |
| Solidification test | | Second | 52 | 25 | 20 | 20 | 120 | 83 | 25 |
| Pellet productivity. | | m/minute | 5.8 | 12.0 | 14.5 | 15.0 | 2.2 | 3.6 | 11.6 |
| Sheet productivity | | m/minute | 2 | 3 | 4 | 4 | (Note 1) | 1 | (Note 2) |
| Number of foreign substances in sheet | | per 100 cm² | 1 | 1 | 2 | 3 | (Note 1) | 25 | (Note 2) |

| | | | | | | | | | |
|---|---|---|---|---|---|---|---|---|---|
| (Note 1) Failure in forming a molded article because of no solidification due to too slow crystallization (Note 2) Failure in forming a molded article because of tear of sheet during sheet forming due to too many unmelted materials (Note 3) The melting point ofPHBH used in the blend is 107°C (Note 4) The melting point ofPHB used in the blend is 173°C | | | | | | | | | |

As shown in Table 1, when the melting point for the compositions of Examples 1 to 4 was measured, the melting point of the second polyhydroxyalkanoate PHB was observed at a temperature lower than the melting point measured for PHB alone. On the other hand, in Comparative Examples 2 to 3, when the melting point for the compositions was measured, the melting point of PHB was observed at the same temperature as the melting point measured for PHB alone.

Further, the PHB content is substantially the same in Example 1 and Comparative Example 2. However, Example 1 was superior to Comparative Example 2 in terms of all of solidification test, pellet productivity, and sheet productivity. Further, Example 4 was also superior to Comparative Example 3 in which PHB content was almost identical to that of Example 4, in terms of all of solidification test, pellet productivity, and sheet productivity.

From the above, it can be seen that the solidification of the resin composition is improved and the processing speed is enhanced at the time of molding accompanied with melting and solidification because the melting point of the second polyhydroxyalkanoate in the resin composition is observed at a temperature lower than the melting point measured for the second polyhydroxyalkanoate alone.

## Claims

1. A polyester resin composition comprising a first polyhydroxyalkanoate and a second polyhydroxyalkanoate, wherein a melting point of the second polyhydroxyalkanoate in the polyester resin composition is observed at a temperature lower than a melting point measured for the second polyhydroxyalkanoate alone.

2. The polyester resin composition according to claim 1, wherein when the resin composition is molded into a 100 µm-thick sheet, the number of foreign substances per 100 cm² of the sheet is 20 or less.

3. The polyester resin composition according to claim 1 or 2, wherein the melting point of the second polyhydroxyalkanoate in the polyester resin composition is observed at a temperature lower by 1 to 50°C than the melting point measured for the second polyhydroxyalkanoate alone.

4. The polyester resin composition according to any one of claims 1 to 3, wherein the first polyhydroxyalkanoate and the second polyhydroxyalkanoate are those that are simultaneously produced by one kind of microorganism.

5. The polyester resin composition according to any one of claims 1 to 4, wherein the second polyhydroxyalkanoate is poly-3-hydroxybutyrate.

6. The polyester resin composition according to any one of claims 1 to 5, wherein the first polyhydroxyalkanoate is at least one selected from the group consisting of poly(3-hydroxybutyrate-co-3-hydroxyhexanoate), poly(3-hydroxybutyrate-co-3-hydroxyvalerate), and poly(3-hydroxybutyrate-co-4-hydroxybutyrate).

7. A molded article comprising the resin composition according to any one of claims 1 to 6.

8. A method for manufacturing the molded article according to claim 7 by processing the resin composition according to any one of claims 1 to 6.
